# EUROPÄISCHE PATENTSCHRIFT

(11) **EP 2 427 150 B1**
(45) Veröffentlichungstag und Bekanntmachung des Hinweises auf die Patenterteilung: **14.08.2013**
(21) Anmeldenummer: 10719269.2
(22) Anmeldetag: 04.05.2010
(51) Int. Cl.: A61F 2/966

(54) **VORRICHTUNG ZUM FREISETZEN EINES SELBSTEXPANDIERBAREN MEDIZINISCHEN FUNKTIONSELEMENTS**
DEVICE FOR RELEASING A SELF-EXPANDABLE MEDICAL FUNCTIONAL ELEMENT
DISPOSITIF POUR RELÂCHER UN ÉLÉMENT FONCTIONNEL MÉDICAL AUTOEXTENSIBLE

(30) Priorität: 05.05.2009 DE 102009020012
(43) Veröffentlichungstag der Anmeldung: 14.03.2012
(73) Patentinhaber: Acandis GmbH & Co. KG, 76327 Pfinztal (DE)
(72) Erfinder: CATTANEO, Giorgio, 76199 Karlsruhe (DE)
(74) Vertreter: Kilchert, Jochen
(86) Internationale Anmeldenummer: PCT/EP2010/002733
(87) Internationale Veröffentlichungsnummer: WO 2010/127838

(56) Entgegenhaltungen:
- EP-A1- 1 374 801
- WO-A1-2007/032809
- US-A- 5 201 757
- US-B1- 6 206 888

## Beschreibung

Die Erfindung bezieht sich auf eine Vorrichtung zum Freisetzen eines selbstexpandierbaren medizinischen Funktionselements mit den Merkmalen des Oberbegriffs des Anspruchs 1.

Eine Vorrichtung der eingangs genannten Art ist beispielsweise aus US 5,201,757 bekannt.

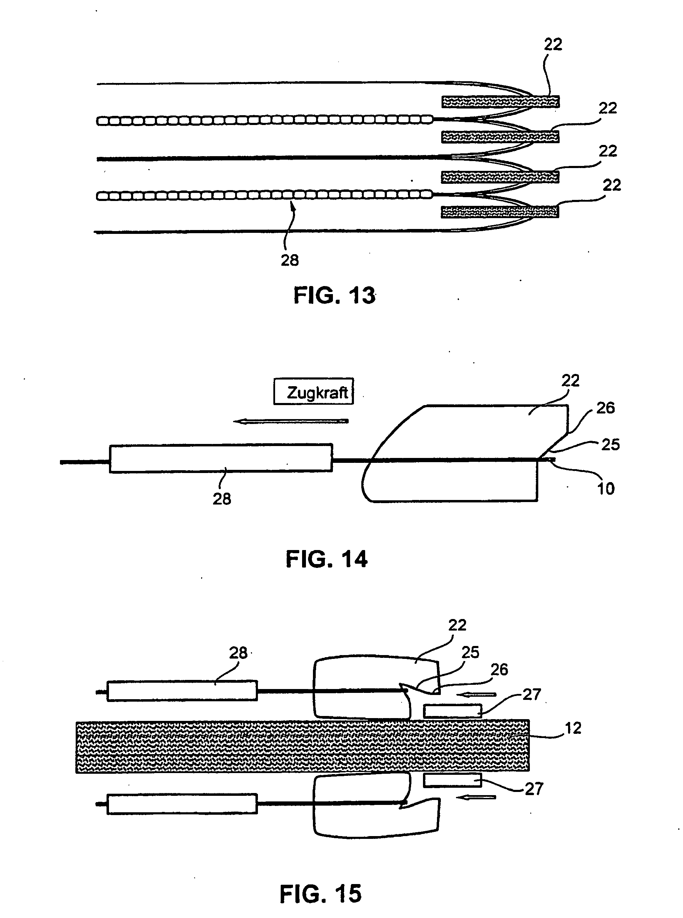

Fig. 13 des US-Patents zeigt einen Einführkatheter zur Implantation selbstexpandierbarer Stents, der eine Radialexpansion des Stents im mittleren Bereich erlaubt, bevor die Stentenden expandieren. Diese Funktion erleichtert das Positionieren des Stents, da der zunächst nur im mittleren Bereich expandierte Stent im Gefäß bewegt werden kann, ohne dabei die Gefaßwände zu verletzen. Dazu weist die bekannte Vorrichtung einen Außenkatheter auf, in dem ein koaxial angeordneter Innenkatheter vorgesehen ist. Der Innenkatheter ist distal mit einer Spitze fest verbunden, die das distale Ende des Stents aufnimmt. Das proximale Ende des Stents ist im Außenkatheter aufgenommen, der von der mit dem Innenkatheter verbundenen Spitze beabstandet ist. Dadurch ist zwischen der mit dem Innenkatheter verbundenen Spitze und dem Ende des Außenkatheters ein Freiraum gebildet, der eine Radialexpansion des mittleren Stentbereichs durch Betätigung des Katheters erlaubt.

Zum Einführen des Stents in das Gefäß ist der Innenkatheter mit einem rohrförmigen Griff verbunden, der zwischen dem Innenkatheter und dem Außenkatheter koaxial angeordnet ist. Zum Setzen des Stents wird der Innenkatheter vom Griff abgeschraubt mit der Folge, dass der Innenkatheter und der Außenkatheter relativ zueinander verschieblich sind. Dadurch kann der Stent im mittleren Bereich in radialer Richtung expandieren, was zu einer Längskontraktion des Stents führt. Aufgrund der Längskontraktion wird die mit dem Innenkatheter verbundene Spitze in Richtung des Außenkatheters gezogen.

Eine Voraussetzung für das Funktionieren dieser Vorrichtung ist die feste Verbindung der Stentenden einerseits mit der Spitze und andererseits mit dem Außenkatheter. Dies erfolgt in beiden Fällen durch Reibschluss zwischen den Stentenden und der jeweiligen Innenwand der Spitze bzw. des Außenkatheters. Die reibschlüssige Verbindung muss so stark sein, dass es zu keiner ungewollten Freisetzung der jeweiligen Stentenden kommt, sondern die gestreckte Anordnung des Stents beibehalten wird, solange der Innenkatheter mit dem Griff verschraubt ist.

Zum Lösen der Stentenden sind daher sowohl in der Spitze als auch im Außenkatheter geeignete Betätigungselemente vorgesehen. In der Spitze ist ein verschiebbarer Anschlag koaxial auf dem Innenkatheter gleitbeweglich angeordnet, der mittels eines Betätigungsdrahtes in proximaler Richtung gezogen werden kann. Dabei nimmt der Anschlag das distale Stentende mit und bewegt dieses aus der Spitze heraus. Im Außenkatheter ist am proximalen Ende des Stents ein Coil vorgesehen, der durch Betätigung des Griffes in distaler Richtung verschiebbar ist, so dass das proximale Stentende aus dem Außenkatheter herausgeschoben werden kann.

Der Aufbau der vorstehend beschriebenen Vorrichtung ist aufwändig und die Handhabung kompliziert, da zum Freisetzen des Stents verschiedene Betätigungsmittel zu aktivieren sind. Außerdem kann ein teilexpandierter Stent nicht mehr in die Vorrichtung wiedereingezogen werden, wenn der Stent beispielsweise vor der endgültigen Platzierung aus dem Gefäß entfernt oder zumindest an eine andere Stelle verschoben werden soll, die im teilexpandierten Zustand des Stents nicht erreichbar ist.

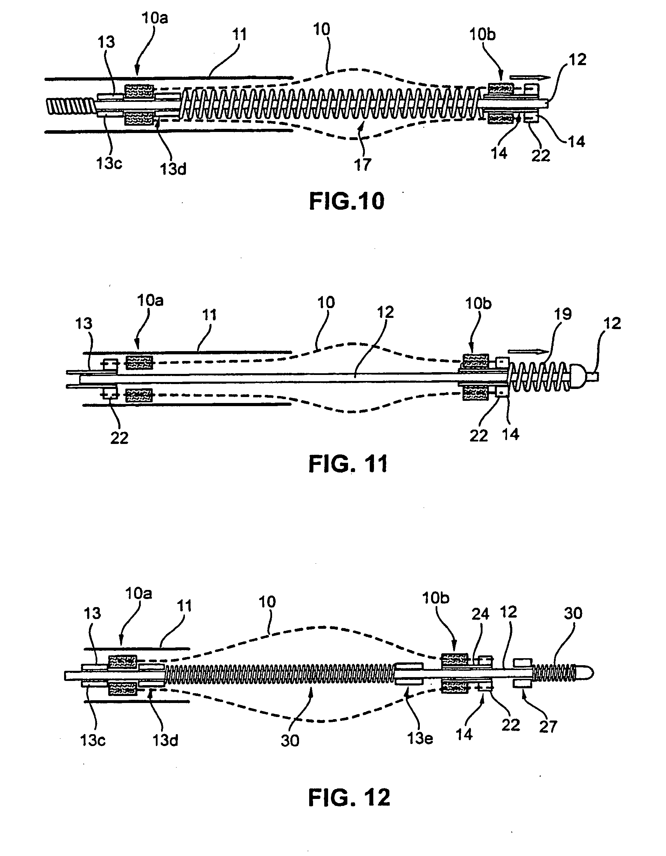

Das US-Patent zeigt in Fig. 10 eine Vorrichtung zum Freisetzen eines Stents, die auf einem anderen Prinzip beruht. Dabei sind die beiden Stentenden jeweils reibschlüssig in zwei in Gegenüberstellung angeordneten Kappen gehalten. Die Kappen sind axial verschieblich auf einem Innenkatheter gelagert, der im Bereich der Kappen gegenläufige Gewinde aufweist, so dass durch Drehung des Innenkatheters die beiden Kappen entweder aufeinander zu oder voneinander weg bewegt werden. Zum Setzen des Stents werden die beiden Kappen so weit voneinander weg bewegt, bis die beiden Stentenden frei sind. Dies setzt voraus, dass sich die beiden Stentenden gleichmäßig lösen, was aber nicht zwingend der Fall ist. Wenn ein Stentende freigesetzt ist und an der Gefäßwand anliegt, wird die gesamte Zugkraft der anderen Kappe über das noch nicht freigesetzte Stentende auf die Gefäßwand übertragen, die dadurch verletzt werden kann.

Eine weitere Vorrichtung zur Implantation eines Stents ist aus WO 2007/098232 A2 bekannt. Diese Vorrichtung umfasst, ähnlich wie die aus US 5,201,757 bekannte Vorrichtung, einen Innen- und Außenkatheter, die zum Freisetzen des Stents relativ zueinander beweglich sind. Koaxial zwischen dem Außen- und Innenkatheter ist ein rohrförmiger Lösemechanismus vorgesehen, der das distale Ende des Stents aus der am Innenkatheter vorgesehenen angebrachten Spitze entfernt. Eine geregelte und kontrollierte Freisetzung der Stentenden soll durch die Einstellung unterschiedlicher Reibungswerte zwischen dem Außenkatheter und dem Lösemechanismus bzw. zwischen dem Innenkatheter und dem Lösemechanismus erfolgen. Aufgrund der verschiedenen relativ zueinander beweglichen Bauteile ist der Aufbau des Katheters aufwändig und überdies fehleranfällig, was sich auf die Sicherheit beim Freisetzen des Stents auswirkt derart, dass eine kontrollierte Freisetzung der Stentenden in Abhängigkeit von den eingestellten Reibungswerten nicht sicher gewährleistet ist.

US 2003/017 6909 A1 offenbart einen Katheter, bei dem ein nach innen umgestülptes Stentende am distalen Ende eines Innenkatheters lösbar befestigt ist. Das proximale Stentende ist im Außenkatheter frei angeordnet. Dieser Katheter erlaubt praktisch kein Einziehen des Stents, nachdem dieser teilweise aus dem Außenkatheter entlassen ist, da es aufgrund der im mittleren Bereich des Stents auftretenden Radialexpansion zur Stauchung des Stents beim Wiedereinziehen kommt.

US 4,655,771 offenbart ein Kathetersystem zum Freisetzen eines Stents, bei dem sowohl das distale als auch das proximale Stentende durch radial bewegbare Verschlusselemente in einer Kappe bzw. im Außenkatheter fixiert sind. Der Aufbau dieses Katheters ist aufgrund der für die Verschlusselemente benötigten Betätigungsmittel aufwändig und fehleranfällig.

Der Erfindung liegt die Aufgabe zugrunde, eine Vorrichtung zum Freisetzen eines selbstexpandierbaren medizinischen Funktionselements, insbesondere eines Stents, anzugeben, die einfach aufgebaut ist und eine gute Positionierbarkeit des Funktionselements ermöglicht.

Erfindungsgemäß wird diese Aufgabe durch die Vorrichtung gemäß Anspruch 1 gelöst.

Die Erfindung beruht darauf, eine Vorrichtung zum Freisetzen eines selbstexpandierbaren medizinischen Funktionselements, insbesondere eines Stents in einem Hohlorgan anzugeben, die eine schlauchförmige Zufuhreinrichtung und ein in der Zufuhreinrichtung axial verschieblich angeordnetes Führungsmittel mit distalen und proximalen Halteelementen aufweist. Die Halteelemente sind mit distalen und proximalen Enden des Funktionselements lösbar verbunden, wobei wenigstens ein Halteelement angepasst ist derart, dass zum Freisetzen wenigstens ein Ende des Funktionselements relativ zum Führungsmittel axial beweglich angeordnet ist. Das proximale und/oder distale Ende des Funktionselements ist zumindest beim Freisetzen mit einer Federkraft beaufschlagt, die der durch die Radialexpansion bewirkten Längskontraktion des Funktionselements entgegenwirkt.

Die Erfindung beruht auf dem erfinderischen Gedanken, die Repositionierbarkeit des Stents, sowohl wenn der Stent die Gefäßwand berührt als auch vor dem Kontakt mit der Gefäßwand, dadurch zu verbessern, dass der Stent ohne die Gefahr einer Stauchung in die Zufuhreinrichtung bei Bedarf wieder vollständig oder zumindest teilweise eingezogen werden kann.

Dieser technische Zusammenhang kommt dadurch zum Ausdruck, dass das proximale und/oder distale Ende des Führungselements zumindest beim Freisetzen mit einer Federkraft beaufschlagt ist, die der durch die Radialexpansion bewirkten Längskontraktion des Führungselements entgegenwirkt. Dadurch wird eine Streckung des Führungselements erreicht, die zur Folge hat, dass die Radialexpansion eines mittleren Bereichs des Funktionselements eingeschränkt ist. Es kommt zu einer verringerten Querschnittsvergrößerung des Führungselements im mittleren Bereich, der somit nicht oder zumindest mit nur einer verringerten Radialkraft an der Gefäßwand anliegt. Das Funktionselement ist damit auch im teilexpandierten Zustand gut positionierbar. Überdies wird durch die Streckung erreicht, dass der Steigungswinkel des Funktionselements im Bereich der mittleren radialen Expansion relativ flach ist, so dass das Funktionselement gut in die Zufuhreinrichtung wieder eingezogen werden kann, ohne dass es zu einer Stauchung kommt. Dabei kann die in proximaler Richtung wirkende Kraft zum Zurückziehen des Funktionselements entweder über die distale Seite des Funktionselements oder über die proximale Seite eingeleitet werden.

Die Vorrichtung gemäß Anspruch 1 hat den weiteren Vorteil, dass diese einen einfachen Aufbau ermöglicht, der eine Freisetzung des Funktionselements im mittleren Bereich gestattet, bevor die Enden des Funktionselements gelöst werden. Zur Betätigung der Vorrichtung ist das Führungsmittel ausreichend. Zusätzliche Betätigungsmittel, die eine Lösefunktion für die Stentenden aufweisen, sind nicht erforderlich.

Bevorzugte Ausführungsformen der Erfindung sind in den Unteransprüchen angegeben.

Das proximale Ende im Bereich der Verbindung mit dem proximalen Halteelement kann durch eine Innenwand der Zufuhreinrichtung und das distale Ende durch das distale Halteelement jeweils in radialer Richtung festgelegt sein. Im Zusammenhang mit den federkraftbeaufschlagten proximalen und distalen Enden bilden diese Merkmale eine bevorzugte Ausführungsform, die durch die Funktionstrennung einen besonders einfachen Aufbau der Vorrichtung ermöglichen. Dabei übernimmt die ohnehin vorgesehene Innenwand der Zufuhreinrichtung die Funktion, das proximale Stentende in radialer Richtung zu fixieren, so dass das proximale Halteelement einfach gestaltet sein kann.

Bei einer weiteren bevorzugten Ausführungsform ist das proximale und distale Halteelement jeweils angepasst derart, dass das proximale und distale Ende durch die Längskontraktion des Funktionselements axial gegeneinander beweglich sind. Die Axialbeweglichkeit beider Enden des Funktionselements ermöglicht eine einfache Lösbarkeit der Enden, wenn der Stent komplett freigesetzt wird.

Das distale Halteelement kann mit dem Führungsmittel verbunden sein derart, dass eine in proximaler und/oder distaler Richtung wirkende Kraft, insbesondere zumindest eine in proximaler Richtung wirkende Kraft durch Betätigung des Führungsmittels auf das Funktionselement übertragbar ist. Damit wird erreicht, dass das Funktionselement durch eine mittels des distalen Halteelements aufgebrachte Zugkraft in die Zufuhreinrichtung wiedereingezogen bzw. allgemein positioniert werden kann.

Alternativ kann das proximale Halteelement mit dem Führungsmittel verbunden sein derart, dass eine in proximaler und/oder distaler Richtung wirkende Kraft, insbesondere zumindest eine in proximaler Richtung wirkende Kraft durch Betätigung des Führungsmittels auf das Funktionselement übertragbar ist. Damit ist das Funktionselement mittels des proximalen Halteelements in die Zufuhreinrichtung wiedereinziehbar bzw. lässt sich generell relativ zur Gefäßwand positionieren.

Zum Strecken des Funktionselements kann eine Druckfeder oder ein federartig wirkendes Element zwischen dem proximalen und distalen Ende angeordnet sein, die zumindest beim Freisetzen mit diesen Enden zusammenwirkt. Damit wird auf einfache Weise eine Beaufschlagung beider Enden mit einer der Längskontraktion entgegengerichteten Federkraft erreicht.

Alternativ oder zusätzlich dazu kann das proximale Halteelement mit einer proximal angeordneten Zugfeder und/oder das distale Halteelement mit einer distal angeordneten Zugfeder jeweils zum Strecken des Funktionselements verbunden sein. Dadurch wird zweckmäßigerweise die Streckkraft erhöht und/oder eine alternative Möglichkeit der Federkrafteinleitung geschaffen.

Das distale und/oder proximale Halteelement kann eine Kappe umfassen, die mit dem Führungsmittel verbunden ist und einen koaxial zum Führungsmittel angeordneten Halte- und Gleitabschnitt aufweist. Der Halte- und Gleitabschnitt fixiert das jeweilige Ende des Funktionselements einerseits in radialer Richtung und führt andererseits in axialer Richtung das jeweilige Ende gleitbeweglich. Damit wird erreicht, dass eine Radialexpansion des Führungselements im mittleren Bereich erfolgt, wobei gleichzeitig das distale Halteelement in radialer Richtung fixiert ist.

Bei einer bevorzugten Ausführungsform umfasst das distale und/oder proximale Halteelement wenigstens einen Flügel, der mit dem Führungsmittel fest oder gleitverschieblich verbunden ist und sich in radialer Richtung erstreckt. Der Flügel ist mit dem jeweiligen Ende des Funktionselements formschlüssig, reibschlüssig oder kraftschlüssig in radialer und axialer Richtung lösbar verbunden. Der Flügel bietet eine Alternative zu dem als Kappe ausgebildeten Halteelement, die besonders für geflochtene Stents bzw. allgemein für geflochtene Funktionselemente geeignet ist, deren Enden Schlaufen aufweisen.

Das proximale und/oder distale Ende des Funktionselements kann mit einer weiteren Federkraft beaufschlagt sein, die in Richtung der Längskontraktion wirkt. Die weitere Federkraft wirkt somit entgegen der Federstreckkraft und dient dazu, die Lösefunktion der Halteelemente zu unterstützen, beispielsweise wenn bei teilweise freigesetztem Funktionselement die entgegen der Längskontraktion wirkende Federstreckkraft nachlässt.

Die Erfindung wird nachfolgend mit weiteren Einzelheiten anhand der beigefügten schematischen Zeichnungen näher erläutert. In diesen zeigen:
- Fig. 1: einen Querschnitt durch eine Vorrichtung nach einem erfindungsgemäßen Ausführungsbeispiel im Ruhezustand mit Streckfeder;
- Fig. 2: die Vorrichtung gemäß Fig. 1 im teilexpandierten Zustand des Funktionselements;
- Fig. 3: die Vorrichtung gemäß Fig. 1 im stärker teilexpandierten Zustand;
- Fig. 4: die Vorrichtung gemäß Fig. 1 im stärker teilexpandierten Zustand;
- Fig. 5: die Vorrichtung gemäß Fig. 1 mit gelöstem proximalen Ende;
- Fig. 6: die Vorrichtung gemäß Fig. 1 mit vollständig expandiertem Funktionselement;
- Fig. 7: einen Querschnitt durch eine Vorrichtung nach einem weiteren erfindungsgemäßen Ausführungsbeispiel mit gelöstem proximalen Ende;
- Fig. 8: einen Querschnitt durch eine Vorrichtung nach einem weiteren erfindungsgemäßen Ausführungsbeispiel im teilexpandierten Zustand des Funktionselements;
- Fig. 9: einen Querschnitt durch eine Vorrichtung nach einem weiteren erfindungsgemäßen Ausführungsbeispiel ohne Funktionselement;
- Fig. 10: einen Querschnitt durch eine Vorrichtung nach einem Ausführungsbeispiel mit proximal fixiertem Halteelement;
- Fig. 11: einen Querschnitt durch eine Vorrichtung nach einem weiteren Ausführungsbeispiel mit proximal fixiertem Halteelement;
- Fig. 12: einen Querschnitt durch eine Vorrichtung nach einem weiteren Ausführungsbeispiel mit proximal fixiertem Halteelement;
- Fig. 13: eine abgewickelt dargestellte Ansicht einer Vorrichtung nach einem erfindungsgemäßen Ausführungsbeispiel im Bereich der Flügel;
- Fig. 14: ein seitlicher Ausschnitt der Vorrichtung gemäß Fig. 13;
- Fig. 15: ein seitlicher Ausschnitt einer Vorrichtung nach einem erfindungsgemäßen Ausführungsbeispiel im Bereich der Flügel;

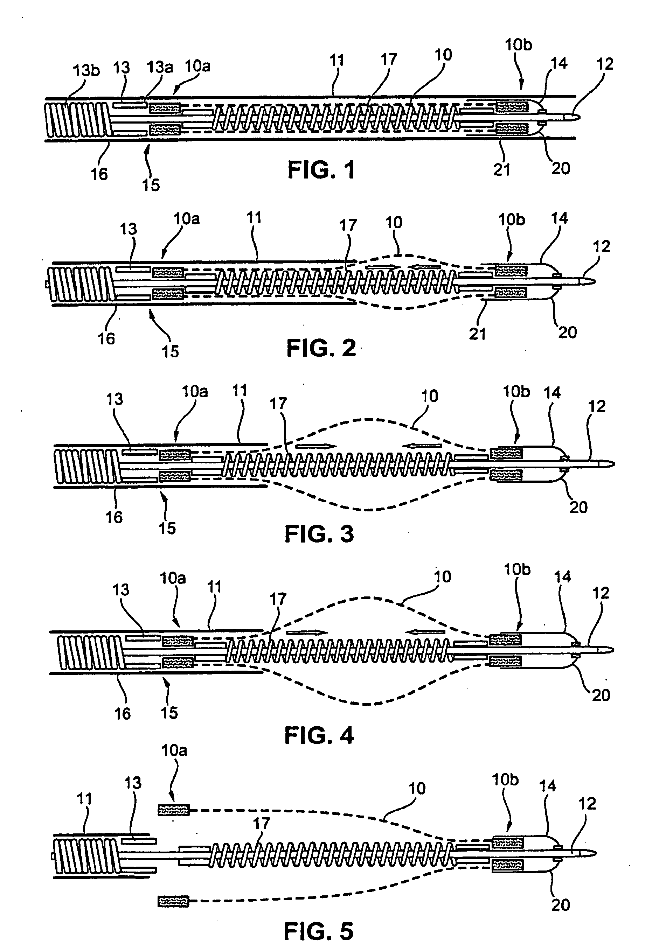

In den Figuren 1-7 ist ein Ausführungsbeispiel für eine Vorrichtung zum Freisetzen eines selbstexpandierbaren medizinischen Funktionselements 10, insbesondere eines Stents dargestellt und zwar in den verschiedenen beim Freisetzen auftretenden Zuständen des Funktionselements bzw. Stellungen der Vorrichtung.

Das Funktionselement 10 bzw. der Stent weist Marker 28 auf, die am proximalen und distalen Stentende 10a, 10b angeordnet sind und radial nach innen und radial nach außen über die Stentkontur hinausragen. Der Stent kann beispielsweise ein geflochtener Stent sein, an dessen Ende die Drähte zusammen verbunden sind. Die Drähte können an den Enden beispielsweise verdrillt sein. Die Erfindung ist auch auf andere Stents, bspw. auf lasergeschnittene Stents mit einem open cell oder einem closed cell design anwendbar.

Das Funktionselement 10 ist nicht auf Stents eingeschränkt, sondern kann andere medizinische Geräte, beispielsweise clot retreiver, Filter und dergleichen umfassen, die in Körperhohlorganen, insbesondere in Blutgefäßen freigesetzt, zumindest temporär freigesetzt werden. Die Vorrichtung ist besonders zum Freisetzen von Stents geeignet.

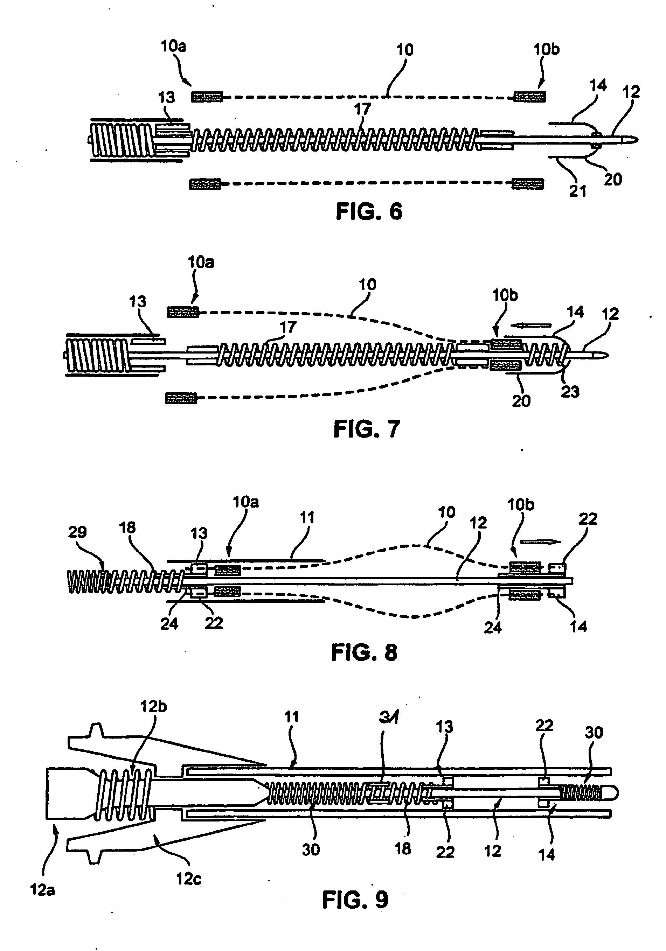

Die Vorrichtung kann ein Katheter, insbesondere ein Mikrokatheter sein. Wie in den Figuren 1 und 9 dargestellt weist die Vorrichtung eine schlauchförmige Zufuhreinrichtung 11, beispielsweise in Form eines Außenkatheters auf. Die Zufuhreinrichtung 11 ist flexibel und kann in ein Gefäß, beispielsweise ein Blutgefäß eingeführt werden. Die Vorrichtung umfasst ein Führungsmittel 12, beispielsweise einen Führungsdraht oder einen Innenkatheter, der in der Zufuhreinrichtung 11 axialverschieblich angeordnet ist. Dazu ist das Führungsmittel 12 mit einem Betätigungsorgan 12a, das mit einer Feder 12b kraftbeaufschlagt sein kann, verbunden. Bei der Feder 12b handelt es sich um eine Kompressionsfeder. Das Betätigungsorgan 12a ist in einem Konnektor, z.B. ein Luer-Anschluss 12c längsaxial geführt. Zwischen dem flexiblen Bereich der Zufuhreinrichtung 11 und dem außerhalb des Katheters angeordneten Betätigungsorgans 12a ist ein Mittelbereich 12d des Führungsmittels 12 bzw. des Führungsdrahts angeordnet, der einen größeren Durchmesser als der im flexiblen Bereich der Zufuhreinrichtung 11 angeordnete Abschnitt des Führungsmittels 12. Der Mittelbereich kann ca. so groß sein wie der Bereich 12 vor dem Stent, wobei der Führungsdraht vor dem Stent aus einem Kern und einer Feder besteht, die dem Führungsdraht die Flexibilität verleiht. Der Außendurchmesser der Feder ist in der Regel ähnlich wie der Außendurchmesser des proximalen Bereiches des Führungsdrahtes. Im Bereich des Stents weist der Führungsdraht in der Regel einen kleineren Durchmesser auf.

Wie in den Figuren 1 und 9 weiter zu erkennen, weist das Führungsmittel 12 proximal und distal angeordnete Halteelemente 13, 14 auf. Der Begriff "proximale" bedeutet näher am Anwender angeordnet und der Begriff "distal" bedeutet weiter entfernt vom Anwender angeordnet.

Damit sind die beiden Haltemittel 13, 14 direkt dem Führungsmittel 12 zugeordnet und werden durch ein einziges bzw. durch dasselbe Führungsmittel 12, also den Führungsdraht betätigt. Dadurch wird die Handhabung und der Aufbau der Vorrichtung wesentlich vereinfacht. Das Führungsmittel 12 dient dabei sowohl zum Platzieren des Funktionselements 12 als auch zum Lösen der Halteelemente 13, 14 vom Funktionselement 10.

Die Haltelemente 13, 14 sind mit den proximalen und distalen Enden 10a, 10b des Funktionselements 10 lösbar verbunden. Das proximale Haltelement 13 ist dazu angepasst, eine in distaler Richtung wirkende Kraft auf das Funktionselement 10 durch Betätigung des Führungsmittels auszuüben (siehe Fig. 1). Die radiale Fixierung des proximalen Endes 10a des Funktionselements 10 erfolgt bei diesem Ausführungsbeispiel durch die Innenwand 16 der Zufuhreinrichtung 10, die das proximale Ende 10a im Bereich der Verbindung 15 zwischen dem proximalen Ende 10a und dem proximalen Halteelement 13 umgibt. Die Funktionstrennung zwischen axialer und radialer Fixierung ermöglicht einen einfachen Aufbau des proximalen Haltelements 13. Beispielsweise kann das proximale Haltelement 13 durch eine Hülse 13a gebildet sein, die an das proximale Ende 10a, insbesondere an die Marker 28 anschlägt und somit lösbar mit diesem verbunden ist. Die Hülse 13a weist einen Außendurchmesser auf, der im Wesentlichen dem Innendurchmesser der Zufuhreinrichtung 11 entspricht und somit den Innenquerschnitt des Katheters füllt. Die Hülse 13a dient somit als Anschlag. Die Hülse 13a steht mit einem proximal davon angeordneten Coil 13b in Verbindung, der am Mittelbereich 12d des Führungsmittels 12 anschlägt, ähnlich wie bei der Vorrichtung gemäß Fig. 9. Eine andere Vergrößerung des Querschnittes des Führungsmittels 12 anstelle des Coils 13b ist möglich. Anstelle der Marker 28 kann der Stent mit seinen Drahtenden direkt am proximalen oder distalen Halteelement 13, 14 anliegen.

Durch diese Anordnung kann eine in distaler Richtung wirkende Kraft auf das Funktionselement 10 ausgeübt werden, das dadurch in distaler Richtung verschoben werden kann, bzw. eine Relativbewegung bezogen auf die Zufuhreinrichtung 10 vollziehen kann.

Das distale Haltelement 14 ist angepasst derart, dass das distale Ende 10b des Funktionselements 10 in radialer Richtung festgelegt ist. Das distale Haltelement 10b kann in die Zufuhreinrichtung 11 einführbar sein und dazu einen etwas kleineren Außendurchmesser als der Innendurchmesser der Zufuhreinrichtung 11 aufweisen. Es ist auch möglich, das distale Halteelement 14 außerhalb der Zufuhreinrichtung 11 anzuordnen.

Bei dem Ausführungsbeispiel gemäß Fig. 1 ist das distale Halteelement 14 als Kappe 20 ausgebildet, die mit dem Führungsmittel 12 fest verbunden ist. Die Kappe 20 ist in proximaler Richtung geöffnet und weist einen koaxial zum Führungsmittel 12 angeordneten Halte- und Gleitabschnitt 21 auf. Der Halte- und Gleitabschnitt 21 ist so in Längsrichtung dimensioniert, dass dieser das distale Ende 10b des Funktionselements 10 aufnimmt und eine gleitbewegliche Führung des distalen Endes 10b bewirkt. Zudem fixiert der Halte- und Gleitabschnitt 21 das distale Ende 10b in radialer Richtung. Der Innendurchmesser des Halte- und Gleitabschnitts 21 ist so bemessen, dass das distale Ende 10b, insbesondere die am distalen Ende 10b angeordneten Marker 28 durch die bei der Radialexpansion entstehenden Längskontraktionskräfte axialverschieblich sind. Der Halte- und Gleitabschnitt 21 fixiert das distale Ende somit in radialer Richtung und ermöglicht eine Bewegung des distalen Endes 10b in axialer Richtung, insbesondere in axialproximaler Richtung. Durch die Länge des Halte- und Gleitabschnitts 21 kann der Zeitpunkt der Freisetzung des distalen Endes 10b eingestellt werden. Insbesondere kann die Länge des Gleitabschnitts 21 so eingestellt werden, dass das distale Ende 10b unmittelbar vor oder nach der Entlassung des proximalen Endes 10a oder gleichzeitig mit der Entlassung des proximalen Endes 10a den Gleitabschnitt 21 verlässt. Die genaue Position, an der sich das distale Ende 10b befindet, wenn das proximale Ende 10a entlassen wird, hängt auch vom Durchmesser des Gefäßes ab, wenn der Mittelbereich des Stents in Kontakt mit der Gefäßinnenwand kommt. Durch Angabe des Gefäßbereiches kann die Länge des Gleitabschnitts 21 sehr genau eingestellt werden.

Zwischen dem distalen Ende 10b und dem proximalen Ende 10a des Funktionselements 10 ist eine Druckfeder 17 bzw. allgemein ein elastisches Element, wie etwa ein elastisches Kunststoffteil, insbesondere ein Silikonschlauch, angeordnet, die zumindest beim Freisetzen eine der Längskontraktion des Funktionselements 10 entgegenwirkende Federkraft auf die beiden Enden 10a, 10b ausübt. Dadurch wird eine Streckung des

Funktionselements 10 erreicht, die die Radialexpansion im mittleren Bereich begrenzt. Die Druckfeder 17 kann im Ruhezustand, das heißt bei nichtexpandiertem Funktionselement 10 entspannt oder vorgespannt sein. Der Außendurchmesser der Feder 17 kann kleiner sein, als der Innendurchmesser der Anschlaghülse 13 bzw. generell des Anschlages, so dass die Feder 17 in der Hülse 13 axial beweglich ist. Damit wird ein verlängerter Entspannungsweg der Feder 17 ermöglicht. Damit kann die Feder 17 vorgespannt sein, während der Stent vollständig gecrimpt ist, wie in Fig. 1 dargestellt. Beim Expandieren des Stents kann aufgrund des verlängerten Entspannungsweges die Vorspannung der Feder 17 vollständig abgebaut werden (Fig. 6). Anstelle der Flügel 22 können Elemente verwendet werden, die einen Vorsprung aufweisen und in die Stentstruktur eingreifen.

Nachfolgend wird die Funktionsweise der Vorrichtung gemäß Fig. 1 näher erläutert:

Im Ruhezustand gemäß Fig. 1 ist der Stent bzw. das Funktionselement 10 vollständig im Katheter bzw. in der Zufuhreinrichtung 11 zusammengefaltet. In dieser Konfiguration ist das Funktionselement 10 am Führungsmittel 12 fixiert und kann sich axial relativ zum Führungsmittel 12 bzw. zum Führungsdraht nicht bewegen. Ein Spiel in axialer Richtung ist auch möglich. Das proximale Ende 10a ist durch das proximale Halteelement 13, insbesondere die Hülse 13a blockiert, die als proximaler Anschlag wirkt. Das distale Ende 10b ist durch das distale Haltelement 14, insbesondere die Kappe 20 blockiert, die einen distalen Anschlag bildet.

Andere Fixierungsmöglichkeiten sind denkbar.

Im Zustand gemäß Fig. 2 ist das distale Ende 10b aus der Zufuhreinrichtung 11 herausgeschoben und es kommt zu einer Auswölbung des Funktionselements 10 radial nach außen. Das distale Ende 10b wird dabei vom distalen Halteelement 14, insbesondere von der Kappe komprimiert gehalten und in radialer Richtung fixiert. Bei der Auswölbung zwischen dem distalen und proximalen Ende 10a, 10b kommt es zu einer Längskontraktion bzw. einer axialen Verkürzung des Funktionselements 10.

Die Druckfeder 17 bewirkt, dass das distale Ende 10b mit dem distalen Halteelement 14 verbunden bleibt, insbesondere nicht aus der Kappe 20 herausrutscht. Die kontinuierlich radiale Fixierung des distalen Stentendes 10b durch den Halte- und Gleitabschnitt 21 der Kappe 20 wird durch eine ausreichende Länge des Halte- und Gleitabschnitts 21 sichergestellt.

Die Druckfeder 17 wird komprimiert, wie in Fig. 2 dargestellt, und sorgt durch die dadurch erzeugte Rückstellkraft, dass die radiale Auswölbung des Funktionselements 10 beschränkt ist. Konkret kommt es zu einem Kräftegleichgewicht zwischen der axial nach Innen gerichteten Druckkraft des Funktionselements 10 aufgrund der axialen Verkürzung einerseits und der entgegengerichteten Rückstellkraft der Feder 17 andererseits. Die Rückstellkraft der Feder 17 führt zu einer axialen Dehnung des Stents oder des Funktionselements 10. Durch eine entsprechende Auslegung der Druckfeder 17 und des Funktionselements 10 ist es möglich, den Grad der Radialexpansion einzustellen, so dass das Funktionselement 10 nicht mit der Gefäßwand in Berührung kommt oder die Gefäßwand nur mit eingeschränkter Kraft streift.

Bei den Zuständen gemäß den Figuren 3, 4 nimmt die Radialexpansion des Funktionselements im mittleren Bereich und die Komprimierung der Druckfeder 17 zu. Das distale Ende 10b gleitet in der Kappe 20 und bleibt dort in radialer Richtung fixiert. In den Zuständen gemäß Figuren 3, 4 ist eine Feinpositionierung des Funktionselements gut möglich, da die Radialexpansion in etwa zentral im mittleren Bereich des Funktionselements 10 erfolgt, so dass eine Ausrichtung des Funktionselements 10 an einem Aneurysma oder an einer Stenose möglich ist. Bei der weitergehenden Radialexpansion des Funktionselements 10 kommt es zu einer im Wesentlichen symmetrischen Verkürzung des Funktionselements 10 an beiden Enden 10a, 10b, so dass die Ausrichtung des Funktionselements 10 an einer Stenose oder einem Aneurisma nicht verändert wird.

Bei der Stellung gemäß Fig. 5 ist das proximale Ende 10a des Funktionselements 10 entlassen. Dies erfolgt dadurch, dass das proximale Haltelement 13, insbesondere die Hülse 13a in distaler Richtung so weit verschoben wird, bis das proximale Ende 10a aus der Zufuhreinrichtung 11 hinausbewegt wird und sich radial entfaltet. Damit lässt die durch die Längskontraktion des Stents 10 auf die Feder 17 ausgeübte Kompressionskraft nach, sodass sich die Feder 17 entspannt. Die auf das distale Element 10b wirkende Federkraft ist damit aufgehoben.

Die Konfiguration gemäß Fig. 6 zeigt das Funktionselement 10 bzw. den Stent in der vollständig entlassenen und vollständig expandierten Stellung. Dazu wird der Führungsdraht 12 mit der Kappe 14 in distale Richtung bewegt, so dass das distale Ende 10b aus der Kappe 20 herausrutscht und vollständig expandiert. Durch eine ausreichende Länge der Kappe 20 bzw. des Halte- und Gleitabschnitts 21 der Kappe 20 wird erreicht, dass das distale Ende 10b nicht unbeabsichtigt oder spontan aus der Kappe 20 herausrutscht. Überdies ist die Ortslage des Stents 10 im Wesentlichen durch die Berührung mit der Gefäßwand fixiert, so dass eine kontrollierte Freisetzung des distalen Endes 10b durch Betätigung des Führungsdrahtes 12 unproblematisch ist, da das distale Ende 310b gleitbeweglich relativ zum Führungsmittel 12 angeordnet ist.

Durch die angepasste Länge des Gleitabschnitts 21 und die plötzlich ausfallende Kraft der Feder 17 kann auch gezielt bewirkt werden, dass das distale Ende 10b beim Entlassen des proximalen Endes 10a automatisch entlassen wird.

Insgesamt ermöglicht die Vorrichtung gemäß den Figuren 1 bis 6 eine Axialbewegung wenigstens eines Endes, insbesondere beider Enden 10a, 10b des Funktionselements 10 relativ zum Führungsmittel 12, wodurch zunächst eine Radialexpansion im mittleren Bereich des Funktionselements 10 erreicht wird. Durch die Relativbewegung zwischen einem bzw. beiden Enden 10a, 10b und dem Führungsmittel 12 wird überdies die Lösefunktion der Vorrichtung ermöglicht, durch die die beiden Enden 10a, 10b aus den jeweiligen Halteelementen 13, 14 gelöst werden. Die Beaufschlagung der beiden Enden 10a, 10b mit einer Federkraft beim Freisetzen, die der Längskontraktion des Führungselements 10 entgegenwirkt, beschränkt einerseits die Radialexpansion im mittleren Bereich des Führungselements 10, wodurch die Positionierbarkeit des Funktionselements verbessert wird. Andererseits bildet die mit der Federkraft einhergehende Streckung des Funktionselements die Voraussetzung dafür, dass das Funktionselement 10 wieder in die Zufuhreinrichtung 11 zurückgeführt werden kann, beispielsweise durch Betätigung des Führungsmittels 12 und des mit dem Führungsmittel 12 verbundenen proximalen oder distalen Halteelements 13, 14. Durch Einziehen des distalen Halteelements 14 wird eine in proximaler Richtung wirkende Zugkraft auf das Funktionselement 10 ausgeübt. Das im teilgestreckten Zustand befindliche Funktionselement 10 kann, ohne gestaucht zu werden, in die Zufuhreinrichtung 11 zurückgezogen werden.

Die Streckung des Funktionselements 10 durch Aufbringen einer Federkraft lässt sich auf verschiedene Vorrichtungen zum Freisetzen von selbstexpandierbaren medizinischen Funktionselementen anwenden.

Beispielsweise kann, wie in Figur 7 dargestellt, das distale Ende 10b mit einer Federkraft beaufschlagt sein, die in Richtung der Längskontraktion, also entgegen der Streckkraft wirkt. Die weitere Federkraft kann beispielsweise durch eine Betätigungsfeder 23 erzeugt werden, die als Druckfeder ausgebildet ist und mit dem distalen Halteelement 14 zusammenwirkt. Konkret kann die Betätigungsfeder 23 in der Kappe 20 distal vom distalen Ende 10b des Funktionselements 10 angeordnet sein. Die Betätigungsfeder 23 bringt eine kleinere Kraft auf, als die im Funktionselement 10 angeordnete Druckfeder 17 derart, dass die Federkraft der Betätigungsfeder 23 zusammen mit der Rückstellkraft des Stents durch die Radialexpansion einerseits und die Federkraft der Druckfeder 17 innerhalb des Funktionselements 10 andererseits so ausgelegt sind, dass es zu einem Kräftegleichgewicht kommt, bevor das Funktionselement 10 aus der Kappe 14 herausgedrückt wird. Damit bleibt das distale Ende 10b des Funktionselements in der Kappe 14, solange die mittig angeordnete Druckfeder 17 komprimiert ist. Wenn das proximale Ende 10a des Funktionselements 10 aus der Zufuhreinrichtung 11 entlassen wird, gibt die Federkraft der Druckfeder 17 nach, so dass die distal angeordnete Betätigungsfeder 23 in der Kappe 14 das distale Ende 10b des Funktionselements aus dieser herausschiebt. Dabei kommt es zu einer Relativbewegung zwischen dem Führungsmittel 12 und dem distalen Ende 10b. Durch dieses System kann verhindert werden, dass die Reibung in der Kappe 14 und in dem Katheter die Bewegung beider Stentenden 10a, 10b bestimmt. Die Feder 23 kann so ausgelegt werden, dass auf jeden Fall das distale Ende 10a sich in proximaler Richtung bewegt und nicht das proximale Ende 10a in distaler Richtung.

Nachfolgend werden mögliche weitere Beispiele für die Halteelemente 13, 14 erläutert.

Wie in Figur 8 dargestellt, kann die Arretierung des distalen Endes 10b des Funktionselements 10 anstelle durch die Kappe 20 durch andere Halteelemente 14 erfolgen. Dasselbe gilt für die proximalen Halteelemente 13. Die im Rahmen der Anmeldung offenbarten konkreten Beispiele für die Ausführung der Halteelemente 13, 14 können kombiniert werden.

Beispielsweise kann die Arretierung des ditalen Endes 10b durch wenigstens einen, insbesondere zwei oder mehr Flügel 22 erfolgen, die sich radial von innen nach außen erstrecken. Die Flügel 22 sind auf dem Führungsmittel 12 angeordnet und entweder fest mit diesem verbunden oder gleitverschieblich gelagert. Bei dem Ausführungsbeispiel gemäß Figur 8 sind die distal angeordneten Flügel 22 fest mit dem Führungsdraht 12 und die proximalen angeordneten Flügel 22 gleitverschieblich auf dem Führungsdraht 12 angeordnet. Umgekehrt ist es auch möglich, die proximalen Flügel 22 fest und die distal angeordneten Flügel 22 gleitverschieblich anzuordnen. Die Flügel 22 sind in den Schlaufen des Funktionselements 10 bzw. des Stents angeordnet. Durch die Rückstellkraft des Stents und die dadurch bewirkte Komprimierung der Streckfeder 17 wird auf die Schlaufen durch die Flügel 22 eine Kraft ausgeübt derart, dass die Schlaufen am Führungsmittel 12 arretiert bleiben. Die Verbindung zwischen den jeweiligen Enden 10a, 10b des Funktionselements 10 und den Flügeln 22 kann formschlüssig, reibschlüssig oder kraftschlüssig sowohl in radialer Richtung als auch in axialer Richtung erfolgen. Die Flügel 22 können auch in Stentzellen eingreifen.

Ein Beispiel für die Ausbildung und Anordnung der Flügel 22 ist in Figuren 13 bis 15 dargestellt. Die Flügel 22 können auf Hülsen 24 befestigt sein, die koaxial auf dem Führungsmittel 12 angeordnet sind, wie in Figur 8 dargestellt. Alternativ können die Flügel direkt auf dem Führungsmittel 12 angeordnet sein (Figur 15). Die Hülse 24 und die radial nach außen gerichteten Flügel 22 können integral aus demselben Teil hergestellt sein. Die Herstellung kann beispielsweise durch ein Spritzgussverfahren, Fräsen, Drah- und/oder Senk-Erosion, Rapid prototyping oder Lasertechnik erfolgen. Die Flügel 22 können auch durch gängige Fügetechniken mit den Hülsen 24 verbunden sein. Die Flügel 22 und die Hülse 24 können einteilig mit dem Führungselement 12, insbesondere dem Führungsdraht, beispielsweise durch Schleifen oder durch Laserprozesse oder andere maschinelle oder chemische Bearbeitungsschritte hergestellt sein.

Die Form der Flügel 22 ist zur Arretierung der Enden 10a, 10b des Funktionselements angepasst. Beispielsweise kann das Profil der einzelnen Flügel 22 so angepasst sein, dass diese eine Arretierung der Schlaufen des Funktionselementes 10 ermöglichen derart, dass die Flügel 22 in der Mitte der einzelnen Schlaufe positioniert sind.

Es ist möglich, die Arretierung durch einen einzigen Flügel 22 oder durch mehrere Flügel 22 vorzunehmen. Bei geflochtenen Stents mit 6, 8, 12 Schlaufen ist eine Arretierung beispielsweise durch 2, 4, 6 oder 12 Flügel 22 vorteilhaft. Bei gelaserten Stents mit 3, 6, 9, 12 Zellen ist die Arretierung durch 3, 6, 9, 12 Flügel, auch in unterschiedlichen Kombinationen, wie z.B. 3 Flügel für 9 Zellen, möglich. Die Flügelarretierung kann auch auf lasergeschnittene Stents bzw. allgemein auf lasergeschnittene Funktionselemente 10 angewendet werden, wobei die vorstehend erwähnte Schlaufe durch eine Zelle, vorzugsweise eine geschlossene Zelle ersetzt wird. Die in den Figuren 13 bis 15 dargestellten Konfigurationen beziehen sich auf einen geflochtenen Stent, dessen Ende durch Schlaufen gebildet sind, ohne auf solche Stents eingeschränkt zu sein. In Figur 13 ist die Anordnung der Schlaufen abgewickelt dargestellt.

In der komprimierten, gecrimpten Konfiguration des Stents liegen die Schlaufen eng beieinander. Im Raum innerhalb einer Schlaufe wird im Gebrauch ein Flügel 22 platziert. Eine kleinere Anzahl an Flügeln 22 verringert den Platzbedarf, wohingegen eine größere Anzahl an Flügeln 22 eine bessere Kraftverteilung bewirkt. Eine optimale Halterung des Funktionselements 10 wird dadurch erreicht, dass jede Schlaufe mit einem Arretierungsflügel 22 versehen ist.

Die einzelnen Flügel 22 weisen jeweils ein Arretierungsprofil auf. Durch eine bei der Radialexpansion entstehende Zugkraft des Funktionselements 10 wird die Schlaufe gegen den Flügel 22 gezogen, so dass die Arretierung sicher bestehen bleibt. Die Richtung der Zugkraft ist in Figur 14 dargestellt. Wie vorstehend beschrieben, herrschen zwei Kräfte, von denen die Konfiguration der Schlaufe, also die Arretierung oder Entlassung des proximalen bzw. distalen Endes 10a, 10b abhängt. Die radiale Rückstellkraft des Funktionselements 10 führt zum Entlassen, der axiale Zug zur Stabilisierung an der Arretierungsstelle. Die radiale Ausweitung des Stents geht mit einer axialen Verkürzung einher, wodurch die Arretierung im Bereich der Flügel 22 weiter stabilisiert wird. Das System kann so ausgelegt werden, dass die Schlaufe erst dann die Arretierungsstelle verlässt, wenn das proximale oder distale Ende 10a, 10b entlassen wird und die axiale Federkraft nachlässt. Die Streckfeder, beispielsweise eine proximal positionierte Zugfeder 18, 19 kann unterschiedliche Federkonstanten aufweisen, wodurch verschiedene Eigenschaften des Systems unterstützt werden. Bei einer hohen Federkonstante ist die Streckung der Feder hoch, was dazu führt, dass das Funktionselement sich wenig ausweitet. Die Arretierung ist in diesem Fall sehr stabil. Bei einer niedrigeren Federkonstante kann sich das Funktionselement 10 weiter radial nach außen ausweiten, wodurch eine Feinpositionierung an einem Aneurysma oder einer Stenose erleichtert wird. Die Streckfeder könnte durch ein anderes steifes Element ersetzt werden, das die Streckung des Funktionselements bewirkt. Das Funktionselement 10 wird bei einem maximalen Streckzustand entsprechend der Konfiguration im Katheter gehalten.

Die Verbindung zwischen dem proximalen und/oder distalen Ende des Funktionselements 10 und dem Flügel 22 kann formschlüssig, reibschlüssig oder kraftschlüssig erfolgen. In den Figuren 14, 15 sind jeweils formschlüssige Verbindungen dargestellt, die einen hinterschnittenen Bereich auf der distalen Seite des jeweiligen Flügels 22 aufweisen. Im Hinterschnitt 25 sind die Schlaufen des Funktionselements 10 angeordnet und somit in radialer und axialer Richtung fixiert. Der Hinterschnitt 25 umfasst einen in distaler Richtung erstreckten Vorsprung 26, der im Gebrauch oberhalb, d.h. radial außen von der Schlaufe bzw. dem Ende 10a, 10b angeordnet ist. Der Vorsprung 26 kann, wie in Figur 14 dargestellt, schräg radial nach außen erstreckt sein, wodurch eine einfache Freisetzung der Schlaufe von dem Flügel 22 ermöglicht wird. Alternativ kann der Vorsprung 26 radial nach innen angeschrägt sein, wie in Figur 15 dargestellt, wodurch eine sichere Arretierung erreicht wird.

Bei dem Ausführungsbeispiel gemäß Figur 15 ist zum Lösen der Arretierung distal von den Flügeln 22 ein Anschlag, insbesondere eine Anschlaghülse 27 angeordnet, die mit dem Führungsmittel 12 fest verbunden ist. Durch eine, durch Pfeile in Figur 15 angedeutete Bewegung des Führungsmittels 12 in proximaler Richtung kann die Anschlaghülse 27 in Anlage mit den Flügeln 22 gebracht werden und nimmt diese in proximaler Richtung mit. Dadurch kommt es zu einer Relativbewegung zwischen den Flügeln 22 und dem jeweiligen Ende 10a, 10b des Funktionselements 10, so dass die Flügel 22 von den Schlaufen des Funktionselements 10 gelöst werden.

Die kraftschlüssige Verbindung könnte beispielsweise durch eine nicht dargestellte Feder gebildet werden, die das Funktionselement gegen den Flügel vorspannt. Zum Lösen der kraftschlüssigen Verbindung kann der in Figur 15 gezeigte Anschlag 27 verwendet werden.

Nachfolgend werden verschiedene Möglichkeiten ausgeführt, die eine Beaufschlagung der Enden 10a, 10b mit einer Federkraft erlauben:

Aus Figur 8 wird deutlich, dass die Beaufschlagung der proximalen und distalen Enden 10a, 10b des Funktionselements 10 mit einer Federkraft auf unterschiedliche Weise erfolgen kann. Beispielsweise kann anstelle oder zusätzlich zu der mittig angeordneten Druckfeder 17 eine proximal mit dem proximalen Halteelement 13 verbundene Zugfeder 18 vorgesehen sein. Die Zugfeder 18 wirkt der Längskontraktion des Funktionselements 10 bei der Radialexpansion entgegen und begrenzt damit die Radialexpansion. Wie in Figur 11 gezeigt, kann das distale Halteelement 14 ebenfalls mit einer Zugfeder 19 verbunden sein, die distal vom distalen Halteelement 14 angeordnet ist. Die Zugfeder 19 ist fest mit dem Führungsdraht 12 verbunden. Die Kombination der beiden Zugfedern 18, 19 mit der mittigen Druckfeder 17 ist möglich.

Die Zugfeder gemäß Figur 8 ist mit dem Führungsmittel 12 fest verbunden, beispielsweise durch eine zwischen der Zugfeder 18 und dem Führungsmittel 12 angeordnete Hülse 24. Die Zugfeder 18 ist proximal mit einem Coil 29 verbunden, der für die Flexibilität des Führungsmittels 12 sorgt.

Wie in Figur 8 weiter dargestellt, ist das distale Ende 10b des Funktionselementes 10 am Führungsmittel 12 fixiert. Das proximale Ende 10a ist axial beweglich durch das proximale Haltelement 13, insbesondere die Flügel 22 gelagert. Die Streckung des Funktionselements erfolgt durch die Zugfeder 18, die in proximaler Richtung in Form des Coils 29 fortgesetzt ist. In Figur 8 ist zu erkennen, dass der Coil 29 am Führungsdraht bzw. Führungsmittel 12 fixiert ist, so dass der Coil 29 nur zur Erhöhung der Flexibilität der Zufuhreinrichtung 11 nicht jedoch zur Federung des proximalen Halteelements 13 beiträgt. Der Bereich zwischen der Fixierungshülse 29 und den Flügeln 22 des Coils 29 ist gestreckt und verhält sich wie eine Feder. Der Zugfederbereich 18 des Coils 29 ist relativ kurz, so dass eine kleine Verlängerung der Feder zu einer großen Federkraft führt. Dadurch wird die radiale Ausweitung des Funktionselements 10 beschränkt und die Streckung und damit auch die Sicherung der Arretierung der distalen Enden 10b verbessert. Zur Vereinfachung des Aufbaus kann die Zugfeder 18 sich über die gesamte Coillänge, also bis zum Bereich 12d des Führungsmittels 12 erstrecken. Die proximalen Flügel 22 können so gestaltet sein, dass die Arretierung nachlässt, wenn sich das proximale Stentende 10b außerhalb des Katheters befindet. Zum Beispiel sind die Flügel 22 in diesem Bereich nicht profiliert oder so schräg profiliert, dass die Entlassung der Schlaufen/Stentzelle erleichtert wird. Beispielsweise kann das Haltemittel 13 mit einer Schräge profiliert sein, die entgegengesetzt gerichtet ist, wie die Schräge des Halteelements 14 bzw. des jeweils entsprechenden Flügels 22. Durch die unterschiedliche Profilierung des proximalen und distalen Flügels 22 wird erreicht, dass der Stent sich automatisch aus der Arretierung löst, wenn dieser freigesetzt ist.

Eine ähnliche Vorrichtung, wie in Figur 8 ist in Figur 9 dargestellt. Bei dieser Vorrichtung ist das distale Halteelement 14 in Form von Flügeln 22 fest mit dem Führungsmittel 12 verbunden. Distal von den Flügeln 22 ist ein Coil 30 angeordnet, der als Marker für die Röntgensichtbarkeit dient. Das proximale Halteelement 13 ebenfalls in Form von Flügeln 22 ist axialbeweglich auf dem Führungsmittel gelagert und mit der Zugfeder 18 verbunden. Die Zugfeder 18 ist durch die Fixierungshülse 31 mit dem Führungsmittel 12 fest verbunden. Zusätzlich ist ein Coil 30 vorgesehen, der sich zwischen der Zugfeder 18 und dem mittleren Bereich 12d des Führungsmittels 12 befindet.

Wie aus Figur 9 hervorgeht, muss die Arretierung des Funktionselements 10 nicht zwangsläufig an den Enden 10a, 10b angreifen. Es ist auch möglich, dass die Arretierung an Stellen angreift, die in Axialrichtung weiter innen, also von den Enden 10a, 10b beastandet angeordnet sind. Aus Figur 9 wird ferner deutlich, wie die Vorrichtung zum Freisetzen eines selbstexpandierbaren medizinischen Funktionselements 10 ohne das geladene Funktionselement 10 aussieht. Dementsprechend wird die Vorrichtung sowohl mit als auch ohne das geladene Funktionselement 10 offenbart und beansprucht und zwar im Zusammenhang mit allen Ausführungsbeispielen dieser Anmeldung.

Anhand der Figuren 10 bis 12 werden Ausführungsbeispiele beschrieben, die ein Beispiel im Hinblick auf das mit dem Führungsmittel 12 verbundene proximale Halteelement 13 verwirklichen, bei dem das Funktionselement 10 durch Betätigung des Führungsmittels 12 zumindest in proximaler Richtung relativ zur Zufuhreinrichtung 11 axialbeweglich ist. Das distale Haltelement 14 ist dabei angepasst derart, dass das distale Ende 10b durch die bei der Radialexpansion bewirkte Längskontraktion des Funktionselements 10 relativ zum Führungsmittel 12 axial beweglich ist. In allen drei Ausführungsbeispielen gemäß Figuren 10 bis 12 ist das proximale Halteelement 13 angepasst derart, dass dieses sowohl in proximaler als auch in distaler Richtung bewegbar ist und dabei das mit dem proximalen Halteelement 13 verbundene proximale Ende 10a des Funktionselements 10 mitnimmt. Das distale Halteelement 14 bzw. allgemein das distale Ende 10b des Funktionselements 10 ist dabei zumindest in proximaler Richtung axialbeweglich.

Das proximale Halteelement 13 weist bei dem Ausführungsbeispiel gemäß Figur 10 zwei Hülsen 13c, 13d auf, die jeweils fest mit dem Führungsdraht 12 verbunden sind und einen in axialer Richtung angeordneten Freiraum bilden, in dem das proximale Ende 10a des Funktionselements 10 angeordnet ist. Dadurch ist das proximale Ende 10a durch die beiden Hülsen 13c, 13d in beiden Axialrichtungen festgelegt. Die radiale Fixierung erfolgt wie beim Ausführungsbeispiel gemäß Figur 1 durch die Innenwand 16 der Zufuhreinrichtung 11. Die mittig angeordnete Druckfeder 17 ist bei dem Ausführungsbeispiel gemäß Figur 10 nicht zwingend erforderlich, um ein Einziehen des Funktionselements 10 in die Zufuhreinrichtung 11 zu ermöglichen. Die Druckfeder 17 verbessert jedoch die Positionierbarkeit, da die Radialexpansion durch die Streckung aufgrund der auf die Enden 10a, 10b ausgeübten Federkraft begrenzt wird. Das distale Halteelement 14 ist axialverschieblich auf dem Führungsmittel 12 gelagert und kann alternativ zu den Flügeln 22 durch die Kappe gemäß Figur 1 gebildet sein.

Bei dem Ausführungsbeispiel gemäß Figur 11 ist das proximale Halteelement 13 in der Form von Flügeln 22 ausgebildet, die fest mit dem Führungsmittel 12 verbunden sind. Das distale Halteelement 14 ist ebenfalls in. Form von Flügeln 22 ausgebildet und durch die Zugfeder 19 in distaler Richtung vorgespannt. Die Zugfeder 19 könnte bei diesem Ausführungsbeispiel entfallen, ohne dass dabei die Gefahr der Stauchung beim Einziehen des Funktionselements 10 in die Zufuhreinrichtung 11 auftritt.

Das Ausführungsbeispiel gemäß Figur 12 verdeutlicht, dass die Streckfeder nicht zwingend erforderlich ist, wenn das proximale Halteelement 13 mit dem Führungsmittel 12 verbunden ist derart, dass das Funktionselement 10 durch Betätigung des Führungsmittels 12 zumindest in proximaler Richtung relativ zur Zufuhreinrichtung 11 axialbeweglich ist. Bei diesem Ausführungsbeispiel ist das proximale Halteelement 13 durch die beiden Hülsen 13c, 13d auf dem Führungsmittel 12 fixiert und erlaubt somit eine Kraftübertragung auf das Funktionselement 10 sowohl in proximaler als auch in distaler Richtung. Das distale Halteelement 14 ist axialbeweglich angeordnet und umfasst Flügel 22, die mit einer gleitverschieblichen Hülse 24 verbunden sind. Die gleitverschiebliche Hülse 24 ist koaxial zum Führungsmittel 12 angeordnet. Zwischen den beiden Halteelementen 13, 14 ist ein Coil 30 angeordnet, der in axialer Richtung bezogen auf das Führungsmittel 12 festgelegt ist. Dazu ist der Coil 30 zwischen der Hülse 13d und einer weiteren distal angeordneten Hülse 13e, die ebenfalls fest mit dem Führungsmittel 12 verbunden ist, angeordnet. Der Coil 30 sorgt für die Flexibilität des Führungsmittels 12 und übernimmt keine Federwirkung. Distal vom distalen Halteelement 14 ist eine Anschlaghülse 27 vorgesehen, die zum Lösen der Arretierung des distalen Halteelements 14 dient. Distal von der Anschlaghülse 27 ist ein weiterer Coil 30 als Röntgenmarker angeordnet. Die im mittleren Bereich des Funktionselements 10 erfolgende Radialexpansion wird durch die axialbewegliche Anordnung des distalen Halteelements 14 in Verbindung mit der Radialexpansion bewirkten Längskontraktion des Funktionselements 10 erreicht. Das proximale Ende 10a ist durch das fixierte proximale Halteelement 13 in axialer Richtung auf dem Führungsmittel 12 festgelegt, so dass das Funktionselement 10 durch Betätigung des proximalen Halteelements 13 positionierbar ist. Dabei kann durch eine Proximalbewegung des Führungsmittels 12 das proximale Halteelement 13 zusammen mit dem proximalen Ende 10a des Funktionselements 10 in die Zufuhreinrichtung 11 eingezogen werden, ohne dass es dabei zu einer Stauchung des Funktionselements 10 kommt.

Bei dem Ausführungsbeispiel gemäß Figur 12 bewegt sich das distale Ende 10b frei auf dem Führungsmittel 12. Die Blockierung des distalen Endes 10b erfolgt durch geometrischen Formschluss, ohne dass dazu eine Kraft in distaler Richtung aufzubringen ist. Wenn das proximale Ende 10a expandiert ist, kann das distale Ende 10b weiterhin arretiert bleiben, bis die Arretierung durch eine proximale Bewegung des Führungsmittels 12 entriegelt wird. Dies erfolgt durch den Anschlag 27. Alternativ könnte die Entriegelung durch eine distale axiale Bewegung des Führungsmittels im Zusammenhang mit einem zum distalen Ende 10b proximal positionierten Anschlag erfolgen, wenn die Arretierung des distalen Endes 10b beispielsweise durch Kappe 14, wie in Figur 1 erfolgt. Dafür könnte der proximal angeordnete Coil 30 mit der Hülse 13c dienen.

Die Arretierung kann bei dem Ausführungsbeispiel gemäß Figur 12 ohne Feder so gestaltet sein, dass sich das arretierte distale Ende 10b nicht ausweitet, wenn sich die Schlaufen während der Entladung des Funktionselementes 10 in proximaler Richtung bewegen. Die Flügel 22 können wie in den Figuren 13 bis 15 profiliert sein. Im Ruhezustand, d.h. ohne oder nur unter Einwirken einer geringen proximal gerichteten Kraft, bleiben die Schlaufen fixiert, auch wenn das proximale Ende 10a bereits entlassen ist. Durch eine nachfolgende proximale Bewegung des Führungsmittels 12 werden die Flügel 22 in proximale Richtung gedrückt. Das Funktionselement 10 ist bereits expandiert und wird von der Gefäßwand festgehalten. Eine leichte Verschiebung der Flügel 22 in proximale Richtung entriegelt damit Arretierung des distalen Endes. Dabei reicht eine kleine Kraft aus, so dass eine Reibung des Funktionselements 10 bzw. des Stents gegen die Gefäßwand vermieden wird.

Offenbart wird ferner eine Vorrichtung zum Freisetzen eines selbstexpandierbaren medizinischen Funktionselements (10), insbesondere eines Stents, in einem Hohlorgan, aufweisend eine schlauchförmige Zufuhreinrichtung (11) und ein in der Zuführeinrichtung (11) axial verschieblich angeordnetes Führungsmittel (12) mit distalen und proximalen Halteelementen (13, 14), die mit distalen und proximalen Enden (10a, 10b) des Funktionselements (10) lösbar verbunden sind, wobei das proximale Ende (10a) im Bereich der Verbindung (15) mit dem proximalen Halteelement (13) durch eine Innenwand (16) der Zufuhreinrichtung (11) und das distale Ende (10b) durch das distale Halteelement (13) jeweils in radialer Richtung festgelegt sind, wobei
das proximale Halteelement (13) mit dem Führungsmittel (12) verbunden ist derart, dass das Funktionselement (10) durch Betätigung des Führungsmittels (12) zumindest in proximaler Richtung relativ zur Zufuhreinrichtung (11) axial beweglich ist, und das distale Halteelement (14) angepasst ist derart, dass das distale Ende (10b) durch die bei der Radialexpansion bewirkte Längskontraktion des Funktionselements (10) relativ zum Führungsmittel (12) axial beweglich ist.

### Bezugszeichenliste

- 10: Funktionselement
- 11: Zufuhreinrichtung
- 12: Führungsmittel
- 13: proximales Halteelement
- 13a: Anschlaghülse
- 13b: Coil
- 13c, 13d: fixierte Hülsen
- 14: distales Halteelement
- 15: Verbindungsstelle
- 16: Innenwand
- 17: Druckfeder
- 18, 19: Zugfeder
- 20: Kappe
- 21: Halte- und Gleitabschnitt
- 22: Flügel
- 23: Betätigungsfeder
- 24: Hülse
- 25: Hinterschnitt
- 26: Vorsprung
- 27: Anschlaghülse
- 28: Marker
- 29, 30: Coil
- 31: Führungshülse

## Patentansprüche

1. Vorrichtung zum Freisetzen eines selbstexpandierbaren medizinischen Funktionselements (10), insbesondere eines Stents, in einem Hohlorgan, aufweisend eine schlauchförmige Zufuhreinrichtung (11) und ein in der Zufuhreinrichtung (11) axial verschieblich angeordnetes Führungsmittel (12) mit distalen und proximalen Halteelementen (13, 14), die mit distalen und proximalen Enden (10a, 10b) des Funktionselements (10) lösbar verbunden sind, wobei wenigstens ein Halteelement (13, 14) angepasst ist derart, dass zum Freisetzen wenigstens ein Ende (10a, 10b) des Funktionselements (10) relativ zum Führungsmittel (12) axial beweglich ist,
**dadurch gekennzeichnet, dass**
das proximale und/oder distale Ende (10a, 10b) des Funktionselements (10) zumindest beim Freisetzen mit einer Federkraft beaufschlagt ist, die der durch die Radialexpansion bewirkten Längskontraktion des Funktionselements (10) entgegenwirkt.

2. Vorrichtung nach Anspruch 1,
**dadurch gekennzeichnet, dass**
das proximale Ende (10a) im Bereich der Verbindung (15) mit dem proximalen Halteelement (13) durch eine Innenwand (16) der Zufuhreinrichtung (11) und das distale Ende (10b) durch das distale Halteelement (14) in radialer Richtung festgelegt sind.

3. Vorrichtung nach Anspruch 1 oder 2,
**dadurch gekennzeichnet, dass**
das proximale und distale Halteelement (13, 14) jeweils angepasst ist derart, dass das proximale und distale Ende (10a, 10b) durch die Längskontraktion des Führungselements (10) axial gegeneinander beweglich sind.

4. Vorrichtung nach wenigstens einem der Ansprüche 1 bis 3,
**dadurch gekennzeichnet, dass** ,
das distale Halteelement (14) mit dem Führungsmittel (12) verbunden ist derart, dass eine in proximaler und/oder distaler Richtung wirkende Kraft durch Betätigung des Führungsmittels (12) auf das Funktionselement (10) übertragbar ist.

5. Vorrichtung nach wenigstens einem der Ansprüche 1 bis 3,
**dadurch gekennzeichnet, dass**
das proximale Halteelement (13) mit dem Führungsmittel (12) verbunden ist derart, dass eine in proximaler und/oder distaler Richtung wirkende Kraft durch Betätigung des Führungsmittels (12) auf das Funktionselement (10) übertragbar ist.

6. Vorrichtung nach wenigstens einem der Ansprüche 1 bis 5,
**dadurch gekennzeichnet, dass**
eine Druckfeder (17) zum Strecken des Funktionselements (10) zwischen dem proximalen und distalen Ende (10a, 10b) angeordnet ist und zumindest beim Freisetzen mit diesen zusammenwirkt.

7. Vorrichtung nach wenigstens einem der Ansprüche 1 bis 6,
**dadurch gekennzeichnet, dass**
das proximale Halteelement (13) mit einer proximal angeordneten Zugfeder (18) und/oder das distale Halteelement (14) mit einer distal angeordneten Zugfeder (19) jeweils zum Strecken des Funktionselements (10) verbunden ist.

8. Vorrichtung nach wenigstens einem der Ansprüche 1 bis 7,
**dadurch gekennzeichnet, dass**
das distale und/oder proximale Halteelement (13, 14) eine Kappe (20) umfasst, die mit dem Führungsmittel (12) verbunden ist und einen koaxial zum Führungsmittel (12) angeordneten Halte- und Gleitabschnitt (21) aufweist, der das jeweilige Ende (10a, 10b) des Funktionselements (10) einerseits in radialer Richtung fixiert und andererseits in axialer Richtung gleitbeweglich führt.

9. Vorrichtung nach wenigstens einem der Ansprüche 1 bis 8,
**dadurch gekennzeichnet, dass**
das distale und/oder proximale Halteelement (13, 14) wenigstens einen Flügel (22) umfasst, der mit dem Führungsmittel (10) fest oder gleitverschieblich verbunden ist und sich in radialer Richtung erstreckt, wobei der Flügel (22) mit dem jeweiligen Ende (10a, 10b) des Funktionselements (10) formschlüssig, reibschlüssig oder kraftschlüssig lösbar verbunden ist.

10. Vorrichtung nach wenigstens einem der Ansprüche 1 bis 9,
**dadurch gekennzeichnet, dass**
das proximale und/oder distale Ende (10a, 10b) des Funktionselements (10) mit einer weiteren Federkraft beaufschlagt ist, die in Richtung der Längskontraktion des Funktionselements (10) wirkt.

## Claims

1. A device for releasing a self-expandable medical functional element (10), in particular a stent, in a hollow organ, comprising a tubular feeder unit (11) and a guiding means (12) arranged to be axially displaceable within the feeder unit (11), having distal and proximal retaining elements (13, 14) releasably connected to distal and proximal ends (10a, 10b) of the functional element (10), wherein at least one retaining element (13, 14) is adapted such that at least one end (10a, 10b) of the functional element (10) is axially movable relative the to guiding means (12) for releasing,
**characterized in that**
at least during the releasing, the proximal and/or distal end (10a, 10b) of the functional element (10) is exposed to a spring force which counteracts the longitudinal contraction of the functional element (10) caused by the radial expansion.

2. The device according to claim 1,
**characterized in that**
in the area of the connection (15) to the proximal retaining element (13), the proximal end (10a) is defined in the radial direction by an interior wall (16) of the feeder unit (11), and the distal end (10b) by the distal retaining element (14).

3. The device according to claim 1 or 2,
**characterized in that**
the proximal and distal retaining elements (14, 14) are each adapted such that the proximal and distal ends (10a, 10b) are axially movable relative to each other by the longitudinal contraction of the guiding element (10).

4. The device according to at least one of claims 1 to 3,
**characterized in that**
the distal retaining element (14) is connected to the guiding means (12) such that a force acting in proximal and/or distal direction may be transmitted to the functional element (10) by actuating the guiding means (12).

5. The device according to at least one of claims 1 to 3,
**characterized in that**
the proximal retaining element (13) is connected to the guiding means (12) such that a force acting in proximal and/or distal direction may be transmitted to the functional element (10) by actuating the guiding means (12).

6. The device according to at least one of claims 1 to 5,
**characterized in that**
a compression spring (17) for extending the functional element (10) is arranged between the proximal and distal ends (10a, 10b) and cooperates with same at least during releasing.

7. The device according to at least one of claims 1 to 6,
**characterized in that**
the proximal retaining element (13) is connected to a proximally arranged tension spring (18) and/or the distal retaining element (14) is connected to a distally arranged tension spring (19), in each case for extending the functional element (10).

8. The device according to at least one of claims 1 to 7,
**characterized in that**
the distal and/or proximal retaining element (13, 14) comprises a cap (20) which is connected to the guiding means (12) and has a holding or sliding portion (21) arranged to be coaxial to the guiding means (12), said holding or sliding portion (21) fixing the respective end (10a, 10b) of the functional element (10) in the radial direction on the one hand and slidably guiding it in the axial direction on the other hand.

9. The device according to at least one of claims 1 to 8,
**characterized in that**
the distal and/or proximal retaining element (13, 14) comprises at least one wing (22) which is connected to the guiding means (10) fixedly or so as to be slidably displaceable and extends in the radial direction, wherein the wing (22) is releasably connected to the respective end (10a, 10b) of the functional element (10) in a form-fitting, frictionally-locking or force-transmitting manner.

10. The device according to at least one of claims 1 to 9,
**characterized in that**
the proximal and/or distal end (10a, 10b) of the functional element (10) is exposed to a further spring force which acts in the direction of the longitudinal contraction of the functional element (10).

## Revendications

1. Dispositif pour libérer un élément fonctionnel médical auto-extensible (10), en particulier un stent, dans un organe creux, présentant un équipement d'acheminement tubulaire (11) et un élément de guidage (12) disposé de façon à être décalé axialement dans l'équipement d'acheminement (11) comportant des éléments de retenue (13, 14) distal et proximal qui sont reliés de façon détachable à des extrémités distale et proximale (10a, 10b) de l'élément fonctionnel (10), dans lequel au moins un élément de retenue (13, 14) est adapté de telle sorte que pour la libération, au moins une extrémité (10a, 10b) de l'élément fonctionnel (10) est mobile axialement par rapport à l'élément de guidage (12),
**caractérisé en ce que**
l'extrémité proximale et/ou distale (10a, 10b) de l'élément fonctionnel (10) reçoit au moins lors de la libération, une force de ressort qui s'oppose à la contraction longitudinale de l'élément fonctionnel (10) entraînée par l'expansion radiale.

2. Dispositif selon la revendication 1,
**caractérisé en ce que**
l'extrémité proximale (10a) est fixée dans la zone de la liaison (15) avec l'élément de retenue proximal (13) par une paroi interne (16) de l'équipement d'acheminement (11) et l'extrémité distale (10b) est fixée par l'élément de retenue (14) distale en direction radiale.

3. Dispositif selon la revendication 1 ou 2,
**caractérisé en ce que**
l'élément de retenue proximal et distal (13, 14) est ajusté respectivement de telle sorte que les extrémités proximale et distale (10a, 10b) soient mobiles axialement l'une par rapport à l'autre, par la contraction longitudinale de l'élément de guidage (10).

4. Dispositif selon au moins l'une des revendications 1 à 3,
**caractérisé en ce que**
l'élément de retenue distal (14) est relié à l'élément de guidage (12) de telle sorte qu'une force agissant en direction proximale et/ou distale puisse être transmise par actionnement de l'élément de guidage sur l'élément fonctionnel (10).

5. Dispositif selon au moins l'une des revendications 1 à 3,
**caractérisé en ce que**
l'élément de retenue proximale (13) est relié à l'élément de guidage (12) de telle sorte qu'une force agissant en direction proximale et/ou distale puisse être transmise par actionnement de l'élément de guidage (12) sur l'élément fonctionnel.

6. Dispositif selon au moins l'une des revendications 1 à 5,
**caractérisé en ce que**
un ressort (17) est disposé pour étirer l'élément fonctionnel (10) entre l'extrémité proximale et distale (10a, 10b) et coopère avec celui-ci au moins lors de la libération.

7. Dispositif selon au moins l'une des revendications 1 à 6,
**caractérisé en ce que**
l'élément de retenue proximal (13) est relié à un ressort de traction (18) disposé sur le plan proximal et/ou l'élément de retenue distal (14) est relié à un ressort de traction disposé sur le plan distal (19) respectivement pour étirer l'élément fonctionnel (10).

8. Dispositif selon au moins l'une des revendications 1 à 7,
**caractérisé en ce que**
l'élément de retenue distal et/ou proximal (13, 14) comprend une coiffe (20) qui est reliée à l'élément de guidage (12) et un segment de retenue et de glissement (21) disposé sur le plan coaxial par rapport à l'élément de guidage (12) qui fixe l'extrémité respective (10a, 10b) de l'élément fonctionnel (10) d'une part en direction radiale et la guide d'autre part en direction axiale par un mouvement de glissement.

9. Dispositif selon au moins l'une des revendications 1 à 8,
**caractérisé en ce que**
l'élément de retenue distal et/ou proximal (13, 14) comprend au moins une ailette (22) qui est reliée fixement à l'élément de guidage (10) ou est reliée de façon à être décalée par glissement et s'étend en direction radiale, l'ailette (22) étant reliée à l'extrémité respective (10a, 10b) de l'élément de fonctionnel (10) par liaison mécanique, liaison par friction ou liaison de force.

10. Dispositif selon au moins l'une des revendications 1 à 9,
**caractérisé en ce que**
l'extrémité proximale et/ou distale (10a, 10b) de l'élément fonctionnel (10) reçoit une autre force de ressort qui agit en direction de la contraction longitudinale de l'élément fonctionnel (10).
